# FASCICULE DE BREVET EUROPEEN

(11) **EP 1 572 989 B1**
(45) Date de publication et mention de la délivrance du brevet: **22.11.2006**
(21) Numéro de dépôt: 03814483.8
(22) Date de dépôt: 16.12.2003
(51) Int. Cl.: C12N 5/06, C12N 5/08, C12Q 1/00, A61K 35/12, A61P 35/00, A61P 37/00

(54) **LIGNEE DE CELLULES DENDRITIQUES GEN2.2**
GEN 2.2 DENDRITISCHE ZELL LINIEN
GEN 2.2 DENDRITIC CELL LINE

(30) Priorité: 16.12.2002 FR 0215927
(43) Date de publication de la demande: 14.09.2005
(73) Titulaire: Etablissement Francais du Sang, 75015 Paris (FR)
(72) Inventeur: PLUMAS, Joel, f-38000 Grenoble (FR); CHAPEROT, Dubonnet, Laurence, f-73800 Francin (FR)
(74) Mandataire: Hinterberg, Katherine
(86) Numéro de dépôt international: PCT/FR2003/003748
(87) Numéro de publication internationale: WO 2004/061089

(56) Documents cités:
- CHAPEROT L., ET AL: "Identification of a leukemic counterpart of the plasmacytoid dendritic ells" BLOOD, vol. 97, no. 10, 15 mai 2001 (2001-05-15), pages 3210-3217, XP002243855 cité dans la demande
- DZIONEK A., ET AL.: "plasmacytoid dentritic cells: from specific surface markers to specific cellular functions" HUMAN IMMUNOLOGY, vol. 63, 2002, pages 1133-1148, XP002243856
- CELLA M. ET AL.: "plasmacytoid monocytes migrate to inflamed lymph nodes and produce large amounts of type I interferon" NATURE MEDICINE, vol. 5, no. 8, août 1999 (1999-08), pages 919-923, XP002243857

## Description

La présente invention concerne des lignées de cellules dendritiques plasmacytoïdes (pDC) humaines, les procédés d'obtention et de culture de ces cellules. L'invention se rapporte également aux utilisations de ces cellules et à des compositions pharmaceutiques comprenant ces cellules dendritiques plasmacytoïdes (pDC) humaines.

Les cellules dendritiques sont des acteurs clés de la réponse immune : elles sont responsables de la capture des antigènes et de leur apprêtement en vue de leur présentation aux lymphocytes T. Différents précurseurs de cellules dendritiques ont été isolés dans le sang, ils expriment HLA DR et CD4, en l'absence de tout autre marqueur spécifique de lignée. Parmi ces précurseurs, Jes mieux caractérisés sont d'origine myéloïde, expriment les molécules CD11c, CD13 et CD33, et se différencient (entre autres) en cellules de Langerhans; ces cellules sont aussi appelées DC1. Une autre population de précurseurs, CD11c-, caractérisés par une très forte expression du récepteur à l'IL3 (CD123) a par ailleurs été identifiée, capable elle aussi de se différencier en cellules dendritiques matures sous l'effet de l'IL3, ou en présence d'un virus, elles sont appelées DC2, ou cellules dendritiques plasmacytoïdes (pDC).

Les pDC, ont été identifiés précisément à partir d'amygdales en 1997 par G. Grouard *et al*. (Grouard G. *et al., J Exp Med.,* 1997;185:1101-1111), elles ont "été aussi décrites dans le sang (O'Doherty U. *et al., Immunology*., 1994;82:487-493 ; Robinson SP. *et al., Eur J Immunol*., 1999;29:2769-2778), dans les ganglions (Cella M. *et al., Nat Med.,* 1999;5:919-923), et le thymus (Res PC. *et al., Blood*., 1999;94:2647-2657 ; Bendriss-Vermare. N. *et al., J Clin Invest.,* 2001;107:835-844). Ces cellules sont caractérisées par leur morphologie de type plasmacytoïde et leur phénotype. Les pDC expriment les molécules CD4, HLA-DR et CD45RA, et sont dépourvues des marqueurs de type myéloïde CD11c et CD13 (Cella M. *et al., Nat Med.,* 1999;5:919-923), ou de marqueurs spécifiques de lignées tels que CD3, CD14 et CD19, bien qu'une expression de CD2, CD5 ou CD7 ait été parfois observée (Cella M. *et al., Nat Med.,* 1999;5:919-923; Res PC. *et al., Blood.,* 1999;94:2647-2657). Plus récemment, la lectine BDCA2 spécifiquement exprimée par les pDC a pu être identifiée ; BDCA4 est retrouvée sur les pDC mais est aussi présente sur les DC dérivées de monocyte (Dzionek A. *et al., J Immunol*., 2000;165:6037-6046 et Human Immunology, vol 63, 2002, pages 1133-1148). Un argument en faveur de l'appartenance de ces cellules à la lignée lymphoïde est leur expression des ARNm codant pour les chaînes preTalpha (Res PC. *et al., Blood*., 1999;94:2647-2657), lambda like 14.1 et SpiB (Bendriss-Vermare N. *et al., J Clin Invest*., 2001;107:835-844). Ces cellules expriment très fortement le récepteur de l'IL-3 et faiblement le récepteur au GM-CSF (Cella M. *et al., Nat Med.,* 1999;5:919-923 ; Rissoan MC. *et al., Science.,* 1999;283:1183-1186), et ces deux cytokines favorisent la survie des pDC (Grouard G. *et al., J Exp Med.,* 1997;185:1101-1111 ; Kohrgruber N. *et al., J Immunol*., 1999;163:3250-3259 ; Robinson SP. *et al., Eur J Immunol*., 1999;29:2769-2778) qui meurent sinon très rapidement *in vitro.* Les molécules de costimulation CD80 et CD86 sont absentes ou faiblement exprimées (Grouard G. *et al., J Exp Med.,* 1997;185:1101-1111), et au stade immature, ces cellules ne sont pas capables d'activer des lymphocytes T (Kohrgruber N. *et al., J Immunol*., 1999;163:3250-3259). En revanche, en présence d'IL-3, de CD40L ou d'un virus, les pDC maturent, expriment fortement les molécules accessoires de la présentation des antigènes (CD40, CD80, CD86 et HLA-DR) et deviennent alors aussi puissantes que des DC d'origine myéloïdes pour activer la prolifération de cellules T allogéniques (Kohrgruber N. *et al., J Immunol*., 1999;163:3250-3259 ; Grabbe S. *et al., Immunol Today.,* 2000;21:431-433 ; Kadowaki N. *et al., J Exp Med.,* 2000;192:219-226). Selon le stimulus responsable de leur maturation (IL3 ou virus), les pDC vont polariser la réponse des lymphocytes T naïfs qu'elles activent, de manière plus ou moins stricte, vers un profil Th1 ou Th2 (Rissoan MC. *et al., Science.,* 1999;283:1183-1186 ; Cella M. *et al., Nat. Immunol*., 2000;1:305-310 ; Kadowaki N. *et al., J Exp Med*., 2000;192:219-226).

Par ailleurs, les pDC au stade immature sont responsables de la sécrétion d'IFN-alpha détectée en réponse aux virus (Cella M. *et al., Nat Med.,* 1999;5:919-923 ; Siegal FP. *et al., Science.,* 1999;284:1835-1837) : elles correspondent aux « interferon producing cells » décrites dans les années 1980. De plus, elles sont capables de répondre aux ADN bactériens et aux produits d'origine virale qu'elles reconnaissent car elles expriment les molécules TLR7 (Ito T. *et al., J Exp Med.,* 2002;195:1507-1512) et TLR9 (Bauer S. *et al., Proc Natl Acad Sci U S A*., 2001;98:9237-9242). Les pDC normales se situent donc à la frontière des réponses immunes innées et adaptatrices, et pourraient avoir un rôle très important dans l'initiation des réponses antivirales.

Les cellules dendritiques plasmacytoïdes (pDC) ouvrent de nouvelles perspectives thérapeutiques car ces cellules sont des acteurs clés de la réponse immune. Les cellules dendritiques jouent ainsi un rôle essentiel dans les défenses immunitaires vis-à-vis d'agents infectieux (bactéries, virus, parasites), dans les défenses immunitaires vis-à-vis des cancers, dans les processus allergiques, dans les réponses autoimmunes, dans l'induction de tolérance et dans l'immunologie des greffes. Les cellules dendritiques sont ainsi utilisées dans différentes applications thérapeutiques. On citera notamment la thérapie cellulaire des cancers (Fong L. et Engleman E., *Annu. Rev. Immunol*., 2000, 18 :245-273).

Un obstacle majeur dans le développement des diverses applications des cellules dendritiques plasmacytoïdes est le problème de l'isolement et de la purification de quantités suffisantes de cellules. En effet, chez l'homme les cellules dendritiques plasmacytoïdes (pDC) représentent moins de 0,5% des cellules circulantes, ce qui rend leur isolement à partir du sang périphérique en grande quantité très difficile. Par ailleurs, les cellules isolées ne prolifèrent pas *in vitro* et meurent rapidement en culture. Ainsi, aucune lignée de cellules dendritiques plasmacytoïdes (pDC) immortalisées n'est disponible à l'heure actuelle. Seule une lignée de cellules dendritiques, d'origine myéloïde et murine, a été décrite (Winzler C. *et al., J Exp Med.,* 1997;185:317-328).

Chaperot *et al*. (Chaperot L. *et al., Blood.,* 2001;97:3210-3217) ont identifié une nouvelle entité de leucémies, impliquant un équivalent tumoral des cellules dendritiques plasmacytoïdes. Ces cellules ont été identifiées lors d'investigations sur des cellules tumorales leucémiques CD4+ CD56+. En effet, ces cellules ne pouvaient être classées dans aucune des catégories de leucémies décrites jusqu'alors. Chaperot *et al*. (Chaperot L. *et al., Blood.,* 2001;97:3210-3217) ont émis l'hypothèse que ces cellules pouvaient appartenir à la lignée des pDC. En effet, mis à part le CD56, le phénotype connu des cellules tumorales était superposable à celui des pDC, en particulier : CD4+, CD11c-, HLADR+, CD123+, CD45RA+. Une étude fonctionnelle a permis de montrer que, comme pour les pDC normales, l'IL3 et le GM-CSF favorisent *in vitro* la survie des cellules tumorales. De plus, ces cellules tumorales expriment les ARNm codant pour les chaînes preTalpha et lambda like 14.1, et en présence de virus elles sont capables de sécréter de l'interféron alpha. De plus, lorsque les cellules tumorales sont cultivées en présence d'IL3, elles subissent une maturation très nette, avec une forte augmentation notamment de l'expression des molécules de costimulation CD40, CD80, CD86, ainsi que l'apparition de CD1a, CD1c et CD83. Cette maturation s'accompagne de l'acquisition de la capacité d'activer des lymphocytes T naïfs, qui sont alors orientés vers un profil de production de cytokines de type Th2. L'identification des pDC leucémiques offre de nouvelles perspectives quant à la caractérisation phénotypique et fonctionnelle des pDC normales. En effet, elles représentent une source de cellules ayant les mêmes caractéristiques que les pDC normales. La pathologie reste cependant rare, 23 patients ayant été identifiés par le GEIL: groupe d'études immunologique des leucémies (Feuillard J. *et al., Blood*., 2002;99:1556-1563), et le recueil de cellules de qualité en grande quantité est très difficile. De plus, les cellules pDC prélevées sur ces patients ne se multiplient pas naturellement *in vitro* et des procédés de mise en culture et de culture *in vitro* de ces cellules n'ont pas été décrits.

La présente invention a donc pour but de proposer un procédé in vitro d'isolement d'une lignée cellulaire de cellules dendritiques plasmacytoïdes (pDC) humaines. Toute lignée cellulaire ainsi obtenue a de nombreuses applications, notamment dans le domaine thérapeutique et dans l'immunologie fondamentale.

### Description de l'invention

La présente invention concerne des lignées de cellules dendritiques plasmacytoïdes humaines de phénotype CD4+, HLA DR +, CD123+, CD45 RA+, CD11c- et CD13-, et leur procédé d'obtention.

Le terme de « lignée cellulaire » s'applique à des cellules de mammifère cultivées *in vitro*. Les cultures primaires de cellules de mammifères ne se multiplient pas en culture ou cessent de se multiplier en culture après un nombre limité de divisions. Les lignées cellulaires selon la présente invention sont capables de se multiplier indéfiniment, ce dont les cultures primaires ou secondaires de cellules de mammifère sont incapables. Ces propriétés des lignées de cellules dendritiques plasmacytoïdes humaines (pDC) selon l'invention permettent d'obtenir avantageusement des quantités importantes de cellules par multiplication ou prolifération de ces cellules *in vitro*. De préférence, les lignées cellulaires selon l'invention sont isolées après au moins 20, 30, 40, 50, 60, 70, 80, 90 et de préférence au moins 100 divisions cellulaires.

Les lignées de cellules dendritiques plasmacytoïdes obtenues selon l'invention peuvent donc être qualifiées d'immortelles puisqu'elles se multiplient indéfiniment *in vitro.*

Les cellules dendritiques plasmacytoïdes propement dites sont connues de l'homme du métier et peuvent être identifiées par leurs caractéristiques morphologiques et par leur phénotype de surface. Ces caractéristiques morphologiques sont un cytoplasme étendu, riche en réticulum endoplasmique et donc basophile avec un noyau excentré ce qui les fait ressembler à des plasmocytes (Siegal, *Science,* 1999, 284:1835-1837, Grouard G. *et al., J Exp Med*., 1997). Ces lignées de cellules dendritiques plasmacytoïdes (pDC) sont également caractérisées par leur phénotype de surface spécifique et notamment par les récepteurs/antigènes exprimés à la surface de ces cellules. Ainsi la distinction des différentes classes de cellules du système immunitaire en fonction des récepteurs/antigènes (marqueurs cellulaires) exprimés à la surface des cellules est une technique largement décrite dans la littérature. Ces analyses du phénotype de surface sont habituellement effectuées par cytométrie en flux. Les cellules dendritiques plasmacytoïdes humaines expriment ainsi notamment les antigènes CD4, HLA-DR, CD123 et CD45 RA. Elles sont dépourvues des marqueurs CD11c et CD13 propres aux cellules dendritiques myéloïdes. Les méthodes d'identification des cellules ayant ce phénotype ont notamment été décrites par Cella M. *et al.* (*Nat Med*., 1999; 5:919-923).

On entend donc par « cellules dendritiques plasmacytoïdes » des cellules ayant les caractéristiques morphologiques requises et un phénotype CD4+, HLA DR +, CD123+, CD45 RA+, CD11c- et CD13-.

Les cellules dendritiques plasmacytoïdes expriment de manière caractéristique les marqueurs cellulaires BDCA2 et BDCA4. Les cellules sont donc de phénotype BDCA2+ et BDCA4+.

La présente invention a pour objet un procédé *in vitro* d'isolement d'une lignée de cellules dendritiques plasmacytoïdes humaines comprenant les étapes de :
a) culture de cellules stromales adhérentes ;
b) mise en culture de cellules tumorales leucémiques d'un patient atteint de leucémie des cellules dendritiques plasmacytoïdes sur lesdites cellules stromales adhérentes dans un milieu de culture approprié; et
c) multiplication des cellules par des divisions cellulaires successives sur lesdites cellules stromales adhérentes dans un milieu de culture approprié, pour obtenir une lignée de cellules dendritiques plasmacytoïdes humaines.

Une entité de leucémie impliquant un équivalent tumoral des cellules dendritiques plasmacytoïdes humaines a été décrite par Chaperot *et al*. (Blood, 2001 ; 97 :3210-3217). De préférence, les cellules tumorales leucémiques mises en culture présentent un phénotype CD4+, HLA DR +, CD123+, CD45 RA+, CD11c- et CD13-. De préférence, ces cellules tumorales sont également de phénotype CD56+. Ce phénotype peut être détecté selon des méthodes connues de l'état de la technique.

De préférence, l'isolement de la lignée s'effectue par mise en culture et culture sur des cellules stromales adhérentes de la lignée murine MS-5.

De préférence, les étapes de mise en culture de cellules tumorales leucémiques et de multiplication des cellules par des divisions cellulaires successives pour obtenir une lignée de cellules dendritiques plasmacytoïdes humaines sont effectuées en présence de cytokines soutenant la prolifération et l'amplification des cellules hématopoïetiques humaines.

Différentes cytokines soutenant la prolifération et l'amplification des progéniteurs et des cellules hématopoïetiques humaines peuvent être utilisées dans les procédés d'isolement d'une lignée de cellules dendritiques plasmacytoïdes (pDC) selon l'invention. Les quantités de cytokines à utiliser dans les procédés selon l'invention sont celles qui sont habituellement utilisées pour les cultures cellulaires *in vitro.*

De préférence, la mise en culture et la multiplication des cellules est effectuée en présence d'au moins une cytokine choisie dans le groupe comprenant IL6, FLT3-L, SCF, IL3, IL7, G-CSF et GM-CSF. Plus préférentiellement, la mise en culture et la multiplication des cellules est effectuée en présence d'au moins une cytokine choisie dans le groupe comprenant le SCF et le FLT3-Ligand.

De préférence, les lignées cellulaires selon l'invention sont établies et isolées après au moins 20, 30, 40, 50, 60, 70, 80, 90 et préférentiellement au moins 100 divisions cellulaires.

Dans un mode de réalisation particulier de l'invention, le procédé *in vitro* d'isolement d'une lignée de cellules dendritiques plasmacytoïdes humaines comprend une étape supplémentaire (d) de clonage de la lignée de cellules dendritiques plasmacytoïdes humaines obtenue à l'étape (c) pour obtenir différentes lignées de cellules dendritiques plasmacytoïdes humaines ou « clones ».

Le « clonage d'une lignée » désigne l'individualisation de cellules de cette lignée, la culture et la multiplication des cellules individualisées pour obtenir des clones ou des lignées de cellules. On entend par « clone » une collection de cellules génétiquement identiques obtenues à partir d'une seule cellule.

Dans un mode de réalisation particulier, le procédé *in vitro* d'isolement d'une lignée de cellules dendritiques plasmacytoïdes humaines comprend donc une étape supplémentaire(e) de sélection desdites différentes lignées ou clones de cellules dendritiques humaines plasmacytoïdes pour identifier les clones présentant un phénotype d'intérêt. Selon un critère d'intérêt, la sélection de variants possédant une caractéristique particulière pourra par exemple permettre d'étudier le rôle d'une molécule ciblée dans la fonction des pDC.

Parmi les différents clones obtenus à partir de la multiplication d'une cellule, on sélectionne ainsi par exemple les clones de cellules dendritiques humaines plasmacytoïdes de phénotype CD56+ ou CD56-.

Un autre objet de la présente invention est une lignée cellulaire isolée susceptible d'être obtenue selon un procédé de l'invention.

Dans un mode de réalisation préféré, l'invention a pour objet la lignée de cellules dendritiques plasmacytoïdes humaines, dénommée GEN2.2, déposée à la CNCM (Collection Nationale de Cultures de Microorganismes, Institut Pasteur, 25 rue du Docteur Roux, F-75015 Paris) le 24 septembre 2002 sous le numéro CNCM I-2938 suivant la règle 6.1 du Traité de Budapest, ou une lignée de cellules dendritiques plasmacytoïdes humaines, dénommée GEN 3, déposée le 16 Octobre 2003 sous le numéro CNCMI-3110 suivant la règle 6.1 du Traité de Budapest.

Un autre objet de la présente invention est un procédé de culture et de multiplication *in vitro* d'une lignée de cellules dendritiques plasmacytoïdes humaines comprenant les étapes de :
a) culture de cellules stromales adhérentes; et
b) culture de la lignée de cellules dendritiques plasmacytoïdes humaines sur lesdites cellules stromales adhérentes dans un milieu de culture approprié.

Les lignées de cellules dendritiques plasmacytoïdes humaines selon l'invention sont capables de se multiplier indéfiniment lorsqu'elles sont cultivées sur des cellules stromales adhérentes. Différentes lignées de cellules stromales adhérentes sont connues de l'homme du métier.

De façon habituelle, la culture des cellules est effectuée dans des flacons, récipients et boites en plastique couramment utilisés dans ce domaine permettant l'adhérence des cellules au support solide. Avantageusement, flacons, récipients et boites en plastique sont « pré-coatés » ou pré-ensemencés avec des cellules stromales adhérentes.

Dans un mode de réalisation particulier, on cultive les cellules stromales jusqu'à confluence.

Dans un mode de réalisation avantageux, les cellules stromales sont ensuite irradiées afin de stopper leur prolifération avant de débuter la culture des lignées de cellules dendritiques plasmacytoïdes humaines sur lesdites cellules stromales.

Les cellules stromales sont connues de l'homme du métier. Il s'agit typiquement de cellules stromales animales ou humaines dérivées de moelle osseuse ou d'autres organes. De préférence, on utilise des cellules stromales adhérentes capables de soutenir la prolifération de cellules progénitrices humaines.

Préférentiellement, les cellules stromales adhérentes sont choisies dans le groupe comprenant les cellules stromales S17, les cellules stromales AFT024, M2-10B4 (ATCC CRL1972), les cellules stromales HESS-5 et les cellules stromales MS-5 (Itoh et al., *Exp.Hematol*., 1989,17 :143-147).

Avantageusement, les cellules stromales adhérentes sont des cellules stromales adhérentes de la lignée murine MS-5 (déposée au DSMZ sous le n° ACC441).

Les milieux de culture pour la culture *in vitro* des lignées de cellules de mammifères sont bien connues de l'homme du métier et couramment utilisés. De préférence, on utilise les milieux de culture habituels tels que du RPMI 1640 Glutamax (GIBCO^{™}) supplémenté par du pyruvate de sodium, des acides aminés non essentiels et du serum de veau foetal décomplémenté.

Un autre objet de la présente invention est un procédé d'obtention *in vitro* de cellules dendritiques plasmacytoïdes humaines activées caractérisé en ce qu'il comprend les étapes suivantes :
a) on dispose d'une lignée cellulaire isolée selon l'invention;
b) on active ladite lignée cellulaire de l'étape a) pour obtenir des cellules dendritiques plasmacytoïdes humaines activées.

Préférentiellement, on active ladite lignée cellulaire par un virus et/ou l'IL3 et/ou le CD40.

Les procédés d'activation *in vitro* d'une lignée de cellules dendritiques plasmacytoïdes humaines selon l'invention permettent d'obtenir en grand nombre des cellules dendritiques plasmacytoïdes activées ou matures. La présente invention se rapporte également à des cellules dendritiques plasmacytoïdes humaines activées ou matures obtenues à partir des lignées cellulaires selon l'invention.

Les procédés d'activation des cellules dendritiques plasmacytoïdes, sont connus de l'homme du métier (Grouard G. *et al., J Exp Med*., 1997; Cella M. *et al., Nat. Immunol*., 2000;1:305-310). L'activation ou la maturation des lignées de cellules dendritiques plasmacytoïdes humaines est donc effectuée selon les techniques habituelles. Contrairement aux procédés de l'état de la technique, les procédés selon la présente invention permettent d'obtenir un grand nombre de cellules activées ou matures grâce à l'utilisation de lignées de cellules dendritiques plasmacytoïdes humaines selon l'invention.

Selon un premier mode de réalisation de l'invention, les lignées cellulaires sont activées par un virus enveloppé ou nu, monocatenaire ou bicatenaire, à ARN (par exemple HIV, HTLV, influenza, oreillons, rougeole, dengue et ebola), à ADN (par exemple adénovirus, HSV, CMV, EBV), ou leurs dérivés (poly-IC), par des bactéries (par exemple *M.tuberculosis*) ou leurs dérivés (CpG ODN), par des parasites (par exemple *leishmanies*) ou des champignons (par exemple *Candida albicans*).

De préférence, l'activation est effectuée en présence d'au moins un virus choisi parmi influenza, HIV et HSV.

Selon un deuxième mode de réalisation, les lignées de cellules dendritiques plasmacytoïdes humaines selon l'invention sont activées par des stimulis d'origine lymphocytaire T qui sont des facteurs solubles comme les cytokines (par exemple IL3, GM-CSF ou IFNα) ou des ligands interagissant avec des récepteurs de surface comme les protéines de la famille du TNF (CD40L ou anti-CD40 par exemple).

Préférentiellement, les lignées de cellules dendritiques plasmacytoïdes humaines selon l'invention sont activées par l'IL3 et/ou le CD40L.

Selon un mode de réalisation particulièrement avantageux, les lignées de cellules dendritiques plasmacytoïdes humaines selon l'invention sont activées par l'IL3, le CD40L et un virus.

A titre d'exemple, l'activation ou la maturation des lignées de cellules dendritiques plasmacytoïdes est induite par l'addition de virus, d'IL3-CD40L ou de virus-IL3-CD40L dans le milieu de culture.

La présente invention concerne également toute lignée cellulaire isolée, dendritique plasmacytoïde humaine, activée, susceptible d'être obtenue selon tout procédé décrit précédemment.

Les cellules dendritiques plasmacytoïdes humaines activées ou matures sont connues de l'homme du métier et peuvent être identifiées ou détectées selon des techniques habituelles.

Typiquement, les cellules dendritiques plasmacytoïdes humaines activées ou matures sécrètent au moins une molécule choisie parmi les cytokines proinflammatoires (par exemple IL6, TNFα et IFNα), les cytokines orientant la réponse immune (par exemple IL12 et IFNα), les chemokines (par exemple IL-8, RANTES, IP10, MIG, MDC, TARC, 1309) et les cytokines anti-virales (par exemple IFNα).

De préférence, les cellules dendritiques plasmacytoïdes humaines activées (ou matures) dérivées de lignées cellulaires selon l'invention sécrètent au moins une molécule choisie parmi l'IL12, le TNF, l'IL6, l'IL8 et l'IFNα.

Habituellement, les cellules dendritiques plasmacytoïdes humaines activées ou matures sont également caractérisées au plan morphologique par de nombreux dendrites et par leur phénotype. La maturation ou l'activation des cellules s'accompagne ainsi d'une forte augmentation de l'expression des molécules HLA et des molécules de costimulation (par exemple CD40, CD80, CD83, CD86) et des récepteurs de chemokines (par exemple CCR6 et CCR7). Ces cellules deviennent alors capables d'activer des lymphocytes T naïfs (Grouard G. *et al., J Exp Med.,* 1997, Cella M. *et al., Nat. Immunol*., 2000;1:305-310, Rissoan MC. *et al., Science.,* 1999;283:1183-1186).

De préférence, les cellules dendritiques plasmacytoïdes humaines activées (ou matures) dérivées de lignées cellulaires selon l'invention expriment au moins un marqueur cellulaire choisi parmi HLA I, CD86, CD80, CCR6 et CCR7 et CD83.

Les cellules dérivées de l'activation ou de la maturation d'une lignée cellulaire de cellules dendritiques plasmacytoïdes selon l'invention présentent au moins une des caractéristiques (sécrétion, morphologie, phénotype, capacité d'activer des lymphocytes T naïfs) des cellules dendritiques plasmacytoïdes activées ou matures.

La présente invention est également relative à un procédé d'activation *in vitro* de lymphocytes T caractérisé en ce qu'il comprend les étapes de :
a) on dispose d'une lignée cellulaire isolée selon l'invention ;
b) on active ladite lignée cellulaire de l'étape a) pour obtenir des cellules dendritiques plasmacytoïdes humaines activées ;
c) on met en contact des lymphocytes T avec lesdites cellules dendritiques plasmacytoïdes humaines activées de l'étape b).

- De préférence, la lignée de cellules dendritiques plasmacytoïdes cellulaire est activée par un virus, l'IL3 et/ou le CD40.

Ce procédé peut être mis en oeuvre sur tout type d'échantillon biologique comprenant des lymphocytes T. De préférence, il s'agit d'un échantillon biologique humain ou animal. L'échantillon est de préférence le sang et pour les applications de type immunothérapie et thérapie cellulaire, il s'agit de sang autologue.

La présente invention se rapporte également à un procédé d'identification de composés activant les cellules dendritiques plasmacytoïdes humaines comprenant les étapes de :
a) mise en contact du composé avec une lignée de cellules dendritiques plasmacytoïdes selon l'invention;
b) détection de l'activation de ladite lignée cellulaire.

La détection de l'activation ou de la maturation des cellules dendritiques plasmacytoïdes humaines est réalisée selon des techniques habituelles connues de l'homme du métier.

Dans un mode de réalisation, on détecte la sécrétion d'au moins une molécule choisie parmi les cytokines proinflammatoires (par exemple IL6, TNFα et IFNα), les cytokines orientant la réponse immune (par exemple IL12 et IFNα), les chemokines (par exemple IL-8, RANTES, IP10, MIG, MDC, TARC, 1309) et les cytokines anti-virales (par exemple IFNα).

De préférence, on détecte la sécrétion d'au moins une molécule choisie parmi l'IL12, le TNF, l'IL6, l'IL8 et l'IFNα.

Dans un autre mode de réalisation, on détecte l'augmentation de l'expression des molécules HLA, des molécules de costimulation (par exemple CD40, CD80, CD86), de CD 83 et des récepteurs de chemokines (par exemple CCR6 et CCR7).

La présente invention concerne également une composition pharmaceutique comprenant au moins une cellule d'une lignée de cellules dendritiques plasmacytoïdes selon l'invention.

Les lignées de cellules dendritiques plasmacytoïdes selon l'invention présentent un intérêt thérapeutique dans le domaine de la réponse immune anti-microbienne et anti-tumorale.

Les lignées de cellules dendritiques plasmacytoïdes selon l'invention peuvent être utilisées dans le traitement de divers types de pathologies, notamment dans le traitement des pathologies liées à des agents infectieux ou microbiens (bactéries, virus, parasites, champignons), des cancers, des allergies et des maladies autoimmunes.

Plus particulièrement, les cellules dendritiques plasmacytoïdes sont capables de présenter des antigènes tumoraux ou microbiens dans des systèmes *in vitro* ou *ex vivo* de façon à induire une réponse immunitaire contre les cellules tumorales ou les cellules infectées.

La présente invention se rapporte plus particulièrement au domaine de la thérapie cellulaire et de l'immunothérapie anti-tumorale. Les lignées de cellules dendritiques plasmacytoïdes selon l'invention peuvent ainsi être utilisées à titre d'agent d'immunothérapie.

Les lignées de cellules dendritiques selon l'invention sont également utilisées pour la fabrication d'une composition pharmaceutique et avantageusement pour la fabrication d'une composition apte à favoriser une réponse immunitaire antitumorale pour le traitement des cancers ou une réponse anti-microbienne pour le traitement des maladies infectieuses.

Les exemples et figures ci-dessous permettront de mettre en évidence certains avantages et caractéristiques de la présente invention.

### Description des figures

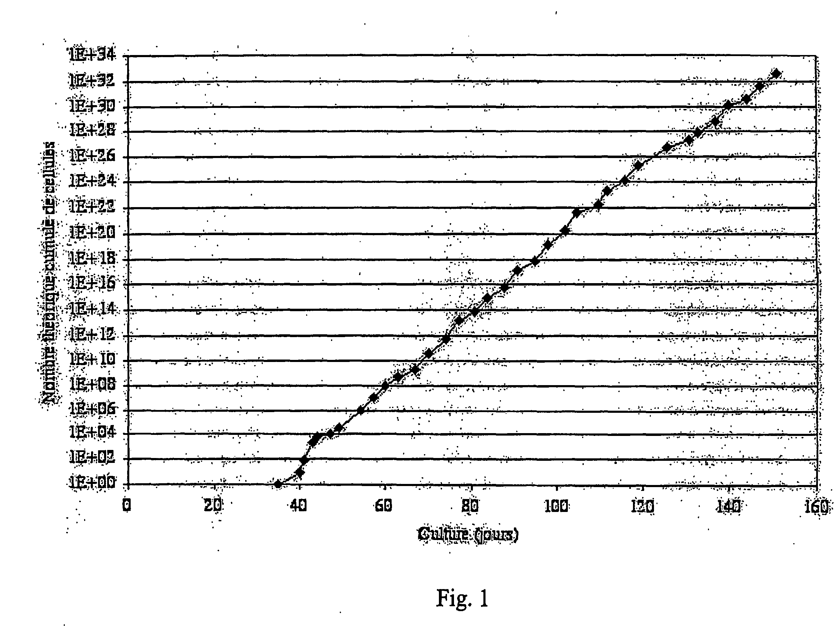

### Figure 1 : Prolifération de la lignée GEN2.2

Après les 35 premiers jours de culture un rythme régulier de prolifération a été obtenu.

Chaque semaine 0,6 millions de cellules GEN2.2 sont co-ensemencées avec des cellules de la lignée MS-5, puis diluées après 3 jours. Deux numérations par semaines sont effectuées. Le nombre cumulé théorique de cellules est calculé à partir de ces numérations.

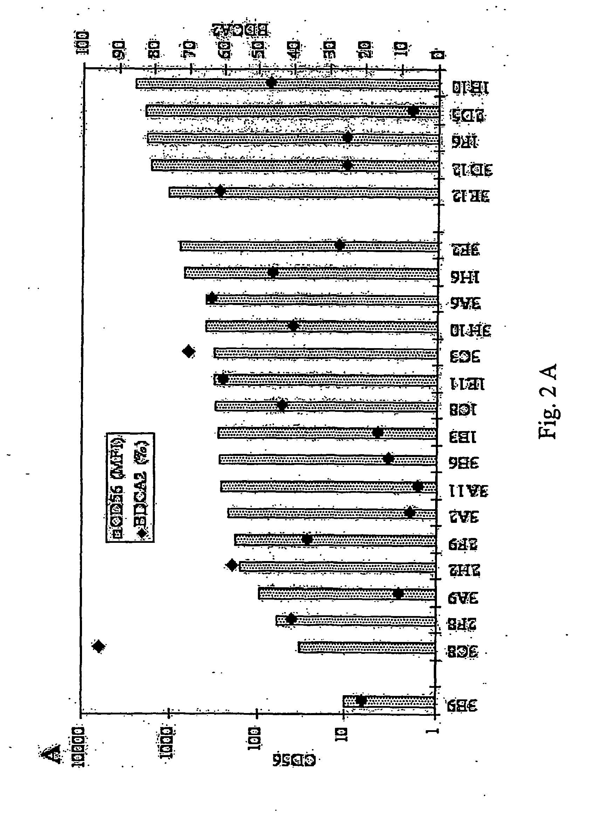

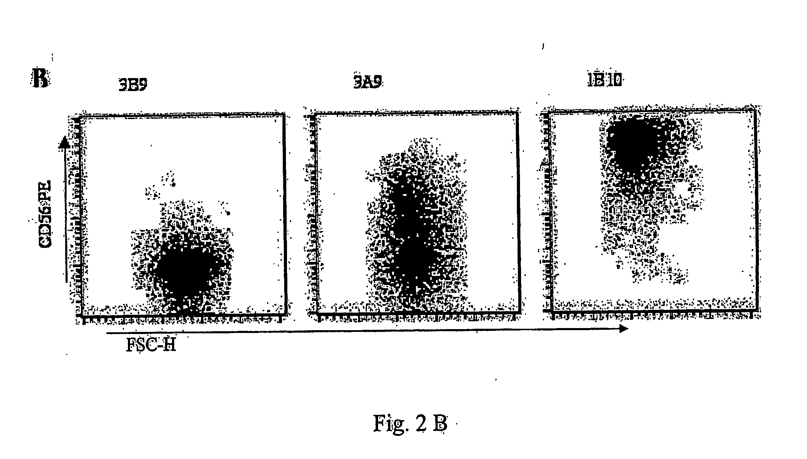

### Figure 2 : Clonage de la lignée GEN2.2

Les 22 sous clones de la lignée GEN2.2 obtenus par dilution limite expriment des niveaux variables de CD56 et BDCA2 (mesure en cytométrie de flux de la moyenne de fluorescence ou du pourcentage de cellules positives) (A). Aucune corrélation n'existe entre les niveaux d'expression de ces deux marqueurs. Parmi les clones étudiés, 1 était négatif pour l'expression de CD56 (3B9), 16 étaient hétérogènes (ex : 3A9), et 5 étaient très positifs (ex : 1B10) (B).

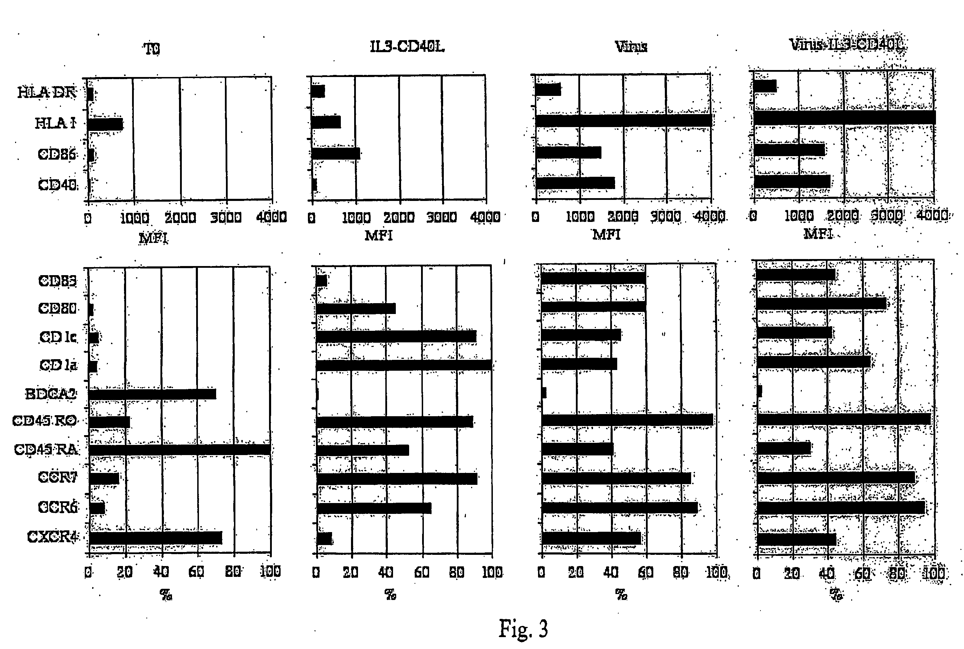

### Figure 3 : Maturation des cellules de la lignée GEN2.2

Les cellules de la lignée GEN2.2 ont été mises en culture durant 48h en présence d'IL3+CD40L, de virus d'influenza ou des trois signaux, puis phénotypées en cytométrie de flux. Dans les trois conditions, une nette maturation des cellules est observée, se traduisant par une augmentation de l'expression des molécules associées aux fonctions de présentation des antigènes (HLA I, CD40, CD80, CD86) (A), et d'autres modifications (augmentation de CCR6 et CCR7, diminution de CXCR4 et BDCA2) (B).

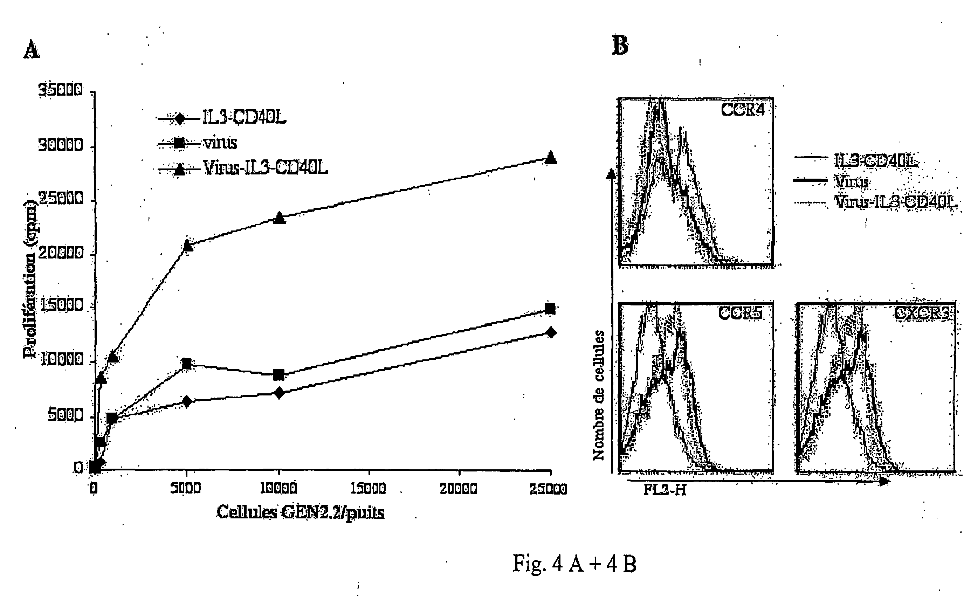

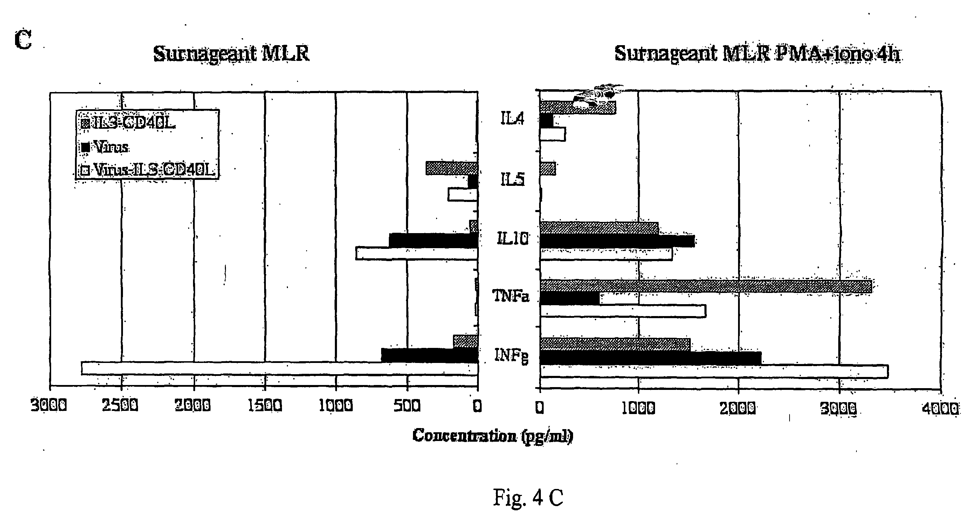

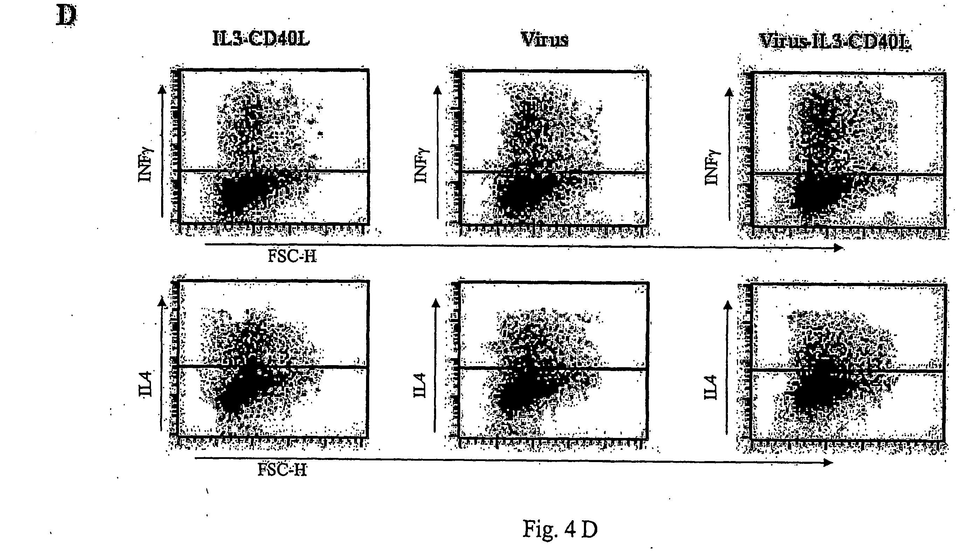

### Figure 4 : Activation et polarisation Th1/Th2 de lymphocytes T naïfs

Après 48h de culture en présence d'IL3+CD40L, de virus ou des trois signaux, les cellules de la lignée GEN2.2 sont capables d'induire la prolifération de lymphocytes T naïfs CD4+ (A).

La prolifération a été mesurée par incorporation de thymidine tritiée sur 18h à la fin des 6 jours de culture mixte. Les lymphocytes T activés au cours de cette MLR expriment CCR4 s'ils ont été activés par les cellules GEN2.2 préactivées en présence d'IL3+CD40L, alors qu'ils expriment CCR5 et CXCR3 lorsqu'ils ont été activés par les cellules de la lignée GEN2.2 préactivées en présence de virus ou virus+IL3+CD40L (B). Les surnageants de la culture mixte et des lymphocytes T réactivés en fin de MLR par PMA+ionomycine présentent plus d'INFg quand les T ont été activés par les cellules de la lignée GEN2.2 préactivées en présence de virus ou virus+IL3+CD40L, alors que plus d'IL4 ou d'IL5 sont retrouvées quand les T ont été activés par les cellules de la lignée GEN2.2 préactivées en présence d'IL3+CD40L (dosage par la technique CBA (Becton Dickinson) en cytométrie) (C). La détection du pourcentage de cellules sécrétant de l'INFg ou de l'IL4 a été réalisée après activation par PMA+ionomycine des lymphocytes T en fin de MLR. Les cellules fixées et perméabilisées sont marquées avec des anticorps spécifiques de ces cytokines et passées au cytomètre (D).

### EXEMPLES

### Exemple 1: Génération d'une lignée humaine de cellules dendritiques plasmacytoïdes

Les cellules pDC leucémiques ont été isolées d'un prélèvement de sang périphérique du patient GEN décrit précédemment (Chaperot L. *et al., Blood*., 2001;97:3210-3217). Les cellules mononucléées isolées qui ont été congelées contenaient plus de 98% de cellules tumorales. La lignée murine stromale adhérente MS-5 (Bennaceur-Griscelli A. *et al., Blood.,* 1999;94:529-538 ; Issaad C. *et al., Blood*., 1993;81:2916-2924) a été utilisée comme « feeder ». Cinq millions de cellules tumorales ont été mises en culture dans un flacon pré-coaté avec des cellules de la lignée MS-5 à confluence, dans 5ml de milieu complet (RPMI 1640 Glutamax (Gibco), supplémenté par 1mM de pyruvate de sodium, 100U/ml de penniciline, 100µg/ml de streptomycine, des acides aminés non essentiels, et 10% de sérum de veau foetal décomplémenté). Durant les cinq premières semaines de la culture du SCF (Nishi N. *et al., Exp Hematol.,* 1996;24:1312-1321) (stem cell factor) et du Flt3-Ligand (Pulendran B. *et al., J Immunol*., 2000;165:566-572 ; Blom B. *et al., J Exp Med.,* 2000;192:1785-1796) ont été ajoutés. Les cellules ont été numérées et diluées avec du milieu frais contenant les cytokines chaque semaine. A ce stade de la culture, une prolifération régulière a été obtenue, et durant les quatre semaines suivantes, 0,6 millions de cellules (alors appelées GEN2) ont été co-ensemencées chaque semaine dans un nouveau flacon avec 0,6 millions de MS-5. Le SCF et le Fit3-L ont alors été supprimés, et la lignée a continué à proliférer de manière satisfaisante sur la sous-couche de MS-5; elle a alors été dénommée GEN2.2. Cette lignée a pu être maintenue pendant au moins 5 mois en culture (**Figure 1**). Le temps de doublement de la lignée est de 1,1 jours. L'irradiation de la sous-couche de MS-5 (60Gy) permet de soutenir de façon identique la prolifération de la lignée GEN2.2, tout en éliminant les MS-5 contaminantes qui pourraient perturber certaines analyses fonctionnelles par leur prolifération. En l'absence de MS-5 les cellules GEN2.2 cessent de proliférer et meurent.

### Exemple 2: Analyse phénotypique et caryotypique de la lignée GEN2.2

Le phénotype des cellules de la lignée GEN2.2 est réalisé en cytométrie de flux, à l'aide d'anticorps directement marqués par FITC ou PE.

Comme les pDC normales, les cellules fraîches de la lignée GEN2.2 sont caractérisées par leur expression de CD4, HLA ABC, HLA DR, CD45RA, CD123 (IL3-Ra), ILT-3, BDCA2 et BDCA4 (tableau 1). CD7 est présent, et 50% des cellules expriment CD56, alors que les autres marqueurs pan-T, B et NK ne sont pas détectés (CD3, CD8, CD19, CD20, CD16, CD57). Les marqueurs myéloïdes (CD13, CD11b, CD11c, CD14, CD64) sont négatifs, alors que le CD33 est positif. En ce qui concerne les molécules de costimulation, les cellules GEN2.2 expriment CD86, 27% sont positives pour CD40, alors que CD80 est absent. CD1a, CD1c et CD83 ne sont pas detectés. Les récepteurs de chémokines/homing tels que CCR1, CCR2, CCR3, CCR4, CCR5, CXCR1, CXCR2, CXCR5 et CLA ne sont pas exprimés, CCR6 et CCR7 sont retrouvés sur 10 à 15% des cellules. Plus de 40% des cellules de la lignée GEN2.2 expriment CXCR3, CXCR4 et CD62L.

**Tableau 1. Phénotype des cellules GEN2.2**

| T/B cells | | | | | | |
|---|---|---|---|---|---|---|
| CD3 | CD4 | CD7 | CD8 | CD19 | CD20 | |
| 0 | 100 | 95 | 2 | 0 | 0 | |

| Myeloid/monocyte | | | | | | |
|---|---|---|---|---|---|---|
| CD13 | CD33 | CD11b | CD11c | CD14 | CD64 | CD116 |
| 2 | 66 | 2 | 10 | 0 | 1 | 25 |

| Natural killer cells | | | | | | |
|---|---|---|---|---|---|---|
| CD16 | CD56 | CD57 | | | | |
| 0 | 51 | 0 | | | | |

| Dendritic cells | | | | | | |
|---|---|---|---|---|---|---|
| CD1a | CD1c | CD83 | BDCA2 | BDCA4 | CD123 | ILT-3 |
| 4 | 5 | 2 | 70 | 88 | 100 | 100 |

| Antigen presenting | | | | | | |
|---|---|---|---|---|---|---|
| cells | | | | | | |
| CD40 | CD80 | CD86 | HLA ABC | HLA DR | | |
| 27 | 2 | 100 | 100 | 100 | | |

| Misceallaneous | | | | | | |
|---|---|---|---|---|---|---|
| CD34 | CD36 | CD38 | CD45RA | CD45RO | CD65 | CD117 |
| 0 | 89 | 74 | 90 | 21 | 17 | 0 |

| Chemokines receptors/Homing | | | | | | |
|---|---|---|---|---|---|---|
| CCR1 | CCR2 | CCR3 | CCR4 | CCR5 | CCR6 | CCR7 |
| 0 | 1 | 5 | 2 | 6 | 8 | 16 |
| CXCR1 | CXCR2 | CXCR3 | CXCR4 | CXCR5 | CLA | CD62L |
| 0 | 6 | 44 | 73 | 1 | 0 | 94 |

Parmi les 11 mitoses analysées, 7 avaient le caryotype suivant 49,XY,+6,t(6:8)(p21;q24),+r(12),+20, et 4 étaient 49,idem,t(3;5)(q21;q21). Ce caryotype est identique à celui de deux sous-clones présents dans les cellules tumorales initiales (patient UPN24) (Leroux D. *et al., Blood.,* 2002;99:4154-4159).

### Exemple 3: sous clonage de la lignée GEN2.2

Une suspension des cellules GEN2.2 a été mise en culture sur une sous-couche de MS-5 confluentes, dans des plaques 96 puits à fond plat, dans 0,2ml de milieu complet. Dans deux plaques (appelées 1 et 2) 0,3 cellules/puits ont été déposées, et dans une plaque (appelée 3) 1 cellule/puits. Après 2 à 5 semaines de culture, les puits proliférants ont été transférés dans des plaques à 24 puits, puis dans des flacons de culture, toujours sur une sous-couche de MS-5.

19/96 puits ont poussé dans la plaque 3, et 12/192 dans les plaques 1 et 2. 22 de ces clones (10 des plaques 1 et 2, et 12 de la plaque 3) ont été amplifiés, phénotypés et congelés. 5 de ces clones expriment fortement la molécule CD56 (plus de 99% de cellules positives, avec une MFI (intensité moyenne de fluorescence)>1000); 1 clone n'exprime pas CD56 (moins de 10% de cellules positives, MFI<10); 16 clones présentent une expression hétérogène de CD56 (49 à 98% de cellules positives, 33<MFI<800) **(figure 2)**. Tous les clones sont positifs pour HLA DR et CD4, et se comportent de la même facon que la lignée GEN2.2 en terme de croissance.

### Exemple 4: Induction de la maturation des cellules GEN2.2 par le virus de l'influenza et/ou l'IL3+CD40L

Les cellules ont été cultivées en milieu complet, en l'absence de MS-5, à 0,5 million/ml. Trois conditions de culture ont été évaluées:
"virus" en présence de virus Influenza (souche A/New Caledonia/20/99, de sous type H1N1, Aventis Pasteur, 137.10⁻³µg d'hémagglutinine/ml),
"IL3-CD40L, par addition d'IL3 (10ng/ml)et de CD40L soluble recombinant (1µg/ml, Alexis),
"virus-IL3-CD40L".

Après 48h de culture, les surnageants de culture ont été congelés pour un dosage d'IFNα par ELISA (Beckman Coulter), et d'IL1-β, IL2, IL4, IL5, IL-6, IL-8, IL-10, IL-12p70, IFN et TNF par les kits TH1/TH2 et cytokines inflammatoires "Cytometric Bead Array" (CBA, BD bioscience). Les cellules ont été récupérées pour un phénotypage en cytométrie, des frottis de cytocentrifugation avec coloration au MGG.

Comme le montre la figure 3, les cellules de la lignée GEN2.2 maturent lorsqu'elles sont mises en culture en présence d'IL3-CD40L, de virus, ou des trois signaux. Cette maturation se traduit par une très nette augmentation des niveaux d'expression des molécules HLA I, CD86 et CD80 qui sont associées aux fonctions de présentation des antigènes. Les molécules HLA DR augmentent aussi, mais dans une moindre mesure. L'expression de CD40 et de CD83 est surtout augmentée dans les deux conditions où le virus est présent. Les cellules GEN2.2 acquièrent aussi les molécules CD1a et CD1c, qui sont connues pour être exprimées par les DC de type myéloïde. Par ailleurs, elles perdent BDCA2 dans toutes les conditions, ainsi que le CXCR4. L'expression de BDCA2 est en effet associée à un stade immature des DC, leur permettant de capter les virus (Dzionek A. *et al., J Exp Med.,* 2001;194:1823-1834) et impliqué dans la régulation de la sécrétion d'IFNα. La perte de CXCR4 dont le ligand est SDF1, une molécule présente dans les organes lymphoïdes secondaires pourrait suggérer que les pDC ne suivent pas les mêmes voies de circulation que les DC myéloïdes pour rejoindre les ganglions, en effet, les MDC n'expriment CXCR4 qu'au stade mature (Sallusto F. *et al., Eur J Immunol*., 1998;28:2760-2769). Au cours de leur maturation, les cellules GEN2.2 acquièrent CCR7 qui pourrait leur permettre de migrer vers les organes lymphoïdes secondaires où elles pourraient rencontrer les lymphocytes T naïf pour les activer, cette up-régulation étant aussi décrite sur les DC d'origine myéloïde (Sallusto F. *et al., Eur J Immunol*., 1998;28:2760-2769). L'inversion du rapport CD45RA/RO observée lors de la maturation de ces cellules, ainsi que l'acquisition du CCR6 sont des données qui ne sont pas encore décrites sur les pDC normales.

Les surnageants des cellules activées par le virus contiennent du TNFα, de l'IL6, de l'IL8 et de l'IFNα. La sécrétion de ces cytokines est nulle ou très faible dans la condition "IL3-CD40L", mais lorsque les trois signaux sont associés, la quantité de TNFα, d'IL6 et d'IL8 détectée est multipliée par trois par rapport à la condition virus (tableau 2), alors que l'IFNα est inchangé, ou un peu diminué. De façon tout à fait intéressante, de l'IL12p70 est présente dans la condition "virus-IL3-CD40L". Cette cytokine joue un rôle très important dans l'activation des lymphocytes Th1 et la différenciation des lymphocytes T cytotoxiques qui sont fondamentaux dans la lutte anti-virale. La production d'IL 12 par les pDC normales a été décrite par certains auteurs (Cella M. *et al., Nat Med.,* 1999;5:919-923 ; Cella M. *et al., Nat. Immunol*., 2000;1:305-310 ; Krug A. *et al., Eur J Immunol*., 2001;31:3026-3037), alors que de nombreuses autres études ont plutôt écrit leur incapacité à produire cette cytokine (Rissoan MC. *et al., Science.,* 1999;283:1183-1186 ; Ito T. *et al., J Exp Med.,* 2002;195:1507-1512 ; Gilliet M. et Liu YJ., *J Exp Med.,* 2002;195:695-704 ; Kadowaki N. *et al., J Exp Med.,* 2001;194:863-869 ; Bauer M. *et al., J Immunol*., 2001;166:5000-5007). Nous n'avons détecté aucune des autres cytokines testées dans les surnageants de ces cellules.

| Tableau 2. Cytokines produites par les cellules GEN2.2 activées | | | | | |
|---|---|---|---|---|---|
| **IL12p70*** | | **TNF*** | **IL6*** | **IL8*** | **IFNα** 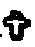 |
| IL3-CD40L | 2 | 45 | 5 | 23 | 0 |
| Virus | 3 | 536 | 2391 | 391 | 1953 |
| Virus-IL3-CD40L | 190 | 1776 | 8690 | 2580 | 1657 |
| * pg/ml, dosage par la technique CBA | | | | | |
| 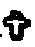 UI/ml, dosage par ELISA | | | | | |

### Exemple 5: Polarisation Th1/Th2 de lymphocytes T naïfs

Les cellules de la lignée GEN2.2 ont été préactivées dans les trois conditions décrites dans l'exemple 4, afin d'évaluer leur capacité à stimuler la prolifération de lymphocytes T naïfs et à induire leur différenciation dans la voie Th1 ou Th2. Les lymphocytes T naïfs CD4+ ont été purifiés à partir de cellules mononucléées de sang de cordon, par une technique immunomagnétique (Stem Cell Technology). Pour la mesure de la prolifération, 25 000 lymphocytes T naïfs par puits ont été stimulés par 25 000, 10 000, 5 000, 1000 et 250 cellules de la lignée GEN2.2 pré-activées. L'incorporation de thymidine tritiée a été mesurée le 6^{ème} jour, sur les 18 dernières heures de la culture. Pour l'évaluation de la polarisation Th1/Th2, 50 000 lymphocytes T naïfs par puits ont été stimulés par 10 000 cellules de la lignée GEN2.2 pré-activées. Après 6 jours de culture, les surnageants de culture ont été congelés, et les cellules récupérées ont été phénotypées (CCR4, CCR5, CXCR3), et activées avec du Phorbol Myristate Acetate (PMA 5ng/ml)+ionomycine (0,5µg/ml) pendant 4 heures ou 6h en présence de monensine (3µM, Sigma) pendant les 4 dernières heures. Un dosage d'IL2, IL4, IL5, IL10, TNFα et IFNγ dans les surnageants avant et après activation par PMA+ionomycine pendant 4h a été réalisé avec le kit CBA Th1/Th2. Les cellules activées par PMA+ionomycine 6h en présence de l'inhibiteur de sécrétion ont été fixées (FACS Lysing, solution, Becton Dickinson), puis perméabilisées (FACS Permeabilizing Solution, Becton Dickinson). La présence de cellules Th1 ou Th2 a été détectée en cytométrie par marquage intracytoplasmique avec des anticorps anti IFNγ et anti IL4.

La plus forte prolifération (25 000 cpm) des cellules T CD4+ naïves a été obtenue avec les cellules pré-activées dans la condition "virus-IL3-CD40L", les cellules pré-activées "IL3-CD40L" et "virus" sont cependant capables d'induire la prolifération des T naïfs (10 000 cpm), confirmant les potentialités APC de type cellule dendritique des cellules de la lignée GEN2.2 **(figure 4A)**. En effet, seules des cellules dendritiques sont capables d'activer efficacement des T naïfs. La polarisation des lymphocytes ainsi activés dans les voies Th1 ou Th2 a été évaluée selon plusieurs critères. Tout d'abord, l'expression de CCR4, décrite comme associée aux Th2 est plus élevée à la surface des T activés par les cellules "IL3-CD40L", alors que l'expression de CCR5 et CXCR3 décrits sur les Th1 est plus forte sur les T activés par les cellules "virus" et "virus-IL3-CD40L" (Sallusto F. *et al., Immunol Today.,* 1998;19:568-574) **(figure 4B).** L'analyse des cytokines dans les surnageants de culture montre que de l'IL5 et de l'IL4 (cytokines Th2) sont produites dans les conditions où les T ont été activés par les cellules "IL3-CD40L" et "virus-IL3-CD40L", alors que l'IFNγ (Th1) est surtout détecté dans les conditions "virus" et "virus IL3-CD40L". De l'IL10 est retrouvée dans tous les cas, pouvant suggérer la présence de lymphocytes régulateurs (Levings MK. *et al., J Exp Med.,* 2001;193:1295-1302; Dieckmann D. *et al., J Exp Med.,* 2001;193:1303-1310) **(figure 4C).** Confirmant ces résultats, la détection des cytokines intracytoplasmiques montre un plus fort pourcentage de cellules productrices d'IFNγ parmi les T activés par les cellules "virus" et "virus-IL3-CD40L", et un plus faible pourcentage de cellules productrices d'IL4 dans la condition "virus" **(figure 4D).**

Ces résultats montrent donc une orientation préférentielle dans la voie Th2 des T naïfs activés par les cellules GEN2.2 "IL3-CD40L", alors qu'un profil plutôt Th1 est induit par les cellules "virus", comme cela a été décrit pour les pDC normales (Rissoan MC. *et al., Science.,* 1999;283:1183-1186 ; Cella M. *et al., Nat. Immunol*., 2000;1:305-310 ; Kadowaki N. *et al., J Exp Med.,* 2000;192:219-226). La condition "virus-IL3-CD40L" induit la plus forte activation des lymphocytes T, les orientant plutôt vers un profil Th1 (CCR5+, CXCR3+, très forte production d'IFNγ), avec cependant la différenciation parallèle de cellules TH2 (production d'IL4 et d'IL5).

### Exemple 6 : lignée cellulaire GEN 3

Les cellules pDC leucémiques ont été isolées d'un prélèvement de sang périphérique du patient GEN décrit précédemment (Chaperot L. e al., Blood,, 2001;97:3210-3217). Les cellules mononucléées isolées qui ont été congelées contenaient plus de 98% de cellules tumorales. La lignée murine stromale adhérente MS-5 (Bennaceur-Griscelli A. et al., Blood,, 1999;94:529-538 ; Issaad C. et al., Blood., 1993 ; 81:2916-2924) a été utilisée comme « feeder » ; elle est utilisée irradiée à 60Gy. Un million de cellules tumorales ont été mises en culture dans un flacon pré-coaté avec 1 million de cellules de la lignée MS-5, dans 5ml de milieu complet (RPMI 1640 Glutamax (Gibco), supplémenté par 1mM de pyruvate de sodium, 100U/ml de penniciline. 100 µg/ml de streptomycine, des acides aminés non essentiels, et 10% de sérum de veau foetal décomplémenté). Les cellules ont été numérées et diluées avec du milieu frais chaque semaine et une prolifération régulière a été obtenue. Cette lignée a pu être maintenue pendant au moins 3 mois en culture. La lignée a été déposée à la CNCM le 16/10/2003 sous le numéro I-3110.

## Revendications

1. Lignée cellulaire constituée de cellules dendritiques plasmacytoïdes humaines immortalisées dénommées GEN 2.2 déposées à la CNCM (Collection Nationale de Cultures de Microorganismes, institut Pasteur, 25 rue du Docteur Roux, F-75015 Paris) le 24 septembre 2002 sous le numéro CNCMI-2938.

2. Lignée cellulaire constituée de cellules dendritiques plasmacytoïdes humaines immortalisées dénommées GEN 3 déposées à la CNCM (Collection Nationale de Cultures de Microorganismes, Institut Pasteur, 25 rue du Docteur Roux, F-75015 Paris) le 16 Octobre 2003 sous le numéro CNCMI-3110.

3. Coculture cellulaire comprenant des cellules de la lignée cellulaire selon l'une quelconque des revendications 1 à 2 et des cellules stromales adhérentes.

4. Procédé d'obtention *in vitro* de cellules dendritiques plasmacytoïdes humaines activées, **caractérisé en ce qu'**il comprend les étapes suivantes :
a) on dispose de cellules selon l'une des revendications 1-2 ;
b) on active les cellules de l'étape a) par des virus ou leurs dérivés, des parasites, des champignons et/ou des stimulis d'origine lymphocytaire T pour obtenir des cellules dendritiques plasmacytoïdes humaines activées.

5. Procédé selon la revendication 4, **caractérisé en ce que** l'on active les cellules par un virus et /ou l'IL3 et/ou le CD40.

6. Procédé d'activation *in vitro* de lymphocytes T, **caractérisé en ce qu'**il comprend les étapes suivantes :
a) on dispose de cellules selon l'une des revendications 1-2 ;
b) on active les cellules de l'étape a) par des virus ou leurs dérivés, des bactéries ou leurs dérivés, des parasites, des champignons et/ou des stimulis d'origine lymphocytaire T pour obtenir des cellules dendritiques plasmacytoïdes humaines activées ;
c) on met en contact des lymphocytes T avec lesdites cellules dendritiques plasmacytoïdes humaines activées de l'étape t).

7. Procédé d'activation *in vitro* de lymphocytes T selon la revendication 6, **caractérisé en ce que** à l'étape b) les cellules sont activées par un virus et /ou l'IL3 et/ou le CD40.

8. Procédé d'identification de composés activant les cellules dendritiques plasmacytoïdes humaines, **caractérisé en ce qu'**il comprend les étapes de :
a) mise en contact, du composé avec des cellules selon l'une des revendications 1-2 ;
b) détection de l'activation des cellules.

9. Composition pharmaceutique **caractérisée en ce qu'**elle comprend des cellules selon l'une des revendications 1-2.

## Claims

1. Cell line consisting in human immortal plasmacytoid dendritic cells called GEN 2.2, deposited with the CNCM (Collection Nationale de Cultures de Microorganismes [National Collection of Cultures of Microorganisms], Pasteur Institute, 25 rue du Docteur Roux, F-75015 Paris) on September 24, 2002, under the CNCM number I-2938.

2. Cell line consisting in human immortal plasmacytoid dendritic cells called GEN 3, deposited with the CNCM (Collection Nationale de Cultures de Microorganismes [National Collection of Cultures of Microorganisms], Pasteur Institute, 25 rue du Docteur Roux, P-75015 Paris) on October 16, 2003, under the CNCM number 1-3110.

3. Cell coculture comprising cells from the cell line according to any of claims 1 to 2 and adherent stromal cells.

4. A method for obtaining activated human plasmacytoid dendritic cells, *in vitro,* **characterized in that** it comprises the following steps:
a) cells as claimed in either of claims 1 and 2 are provided;
b) the cells of step a) are activated with viruses or derivatives thereof, parasites, fungi and/or stimuli of T lymphocyte origin, so as to obtain activated human plasmacytoid dendritic cells.

5. The method as claimed in claim 3, **characterized in that** the cells are activated with a virus and/or IL3 and/or CD40.

6. A method for activating T lymphocytes, *in vitro*, **characterized in that** it comprises the following steps:
a) cells as claimed in either of claims 1 and 2 are provided;
b) the cells of step a) are activated with viruses or derivatives thereof, bacteria or derivatives thereof, parasites, fungi and/or stimuli of T lymphocyte origin, so as to obtain activated human plasmacytoid dendritic cells;
c) T lymphocytes are brought into contact with said activated human plasmacytoid dendritic cells of step b).

7. The method for activating T lymphocytes, *in vitro*, as claimed in claim 6, **characterized in that**, in step b), the cells are activated with a virus and/or IL3 and/or CD40.

8. A method for identifying compounds that activate human plasmacytoid dendritic cells, **characterized in that** it comprises the steps consisting in:
a) bringing the compound into contact with cells as claimed in either of claims 1 and 2;
b) detecting the activation of the cells.

9. A pharmaceutical composition, **characterized in that** it comprises cells as claimed in either of claims 1 and 2.

## Patentansprüche

1. Zelllinie, die aus immortalisierten, humanen, plasmazytoiden, dendritischen Zeilen besteht, die die Bezeichnung GEN 2.2 haben und am 24. September 2002 unter der Nummer CNCMI-2938 bei der CNCM (Collection Nationale de Cultures de Microorganismes, Institut Pasteur, 25 rue du Docteur Roux, F-75015 Paris) hinterlegt wurden.

2. Zelllinie, die aus immortalisierten, humanen, plasmazytoiden, dendritischen Zellen besteht, die die Bezeichnung GEN 3 haben und am 16. Oktober 2003 unter der Nummer CNCMI-3110 bei der CNCM (Collection Nationale de Cultures de Microorganismes, Institut Pasteur, 25 rue du Docteur Roux, F-75015 Paris) hinterlegt wurden.

3. Cozellkultur, die Zellen der Zelllinie gemäß einem der Ansprüche 1 bis 2 und adhärente Strornazellen aufweist.

4. Verfahren, um aktivierte, humane, plasmazytoide dendritische Zellen *in vitro* zu erhalten, **dadurch gekennzeichnet, dass** es die folgenden Schritte aufweist;
a) Bereitstellen von Zellen gemäß einem der Ansprüche 1 bis 2;
b) Aktivieren der Zellen aus Schritt a) durch Viren oder deren Abkömmlinge, durch Parasiten, durch Pilze und/oder durch Stimuli von T-lymphozytärem Ursprung, um aktivierte, humane, plasmazytoide, dendritische Zellen zu erhalten.

5. Verfahren nach Anspruch 4, **dadurch gekennzeichnet, dass** die Zellen durch ein Virus und/oder durch IL3 und/oder durch CD40 aktiviert werden.

6. Verfahren, um T-Lymphozyten *in vitro* zu aktivieren, **dadurch gekennzeichnet, dass** es die folgenden Schritte aufweist:
a) Bereitstellen von Zellen gemäß einem der Ansprüche 1 bis 2;
b) Aktivieren der Zellen aus Schritt a) durch Viren oder deren Abkömmlinge, durch Bakterien oder deren Abkömmlinge, durch Parasiten, durch Pilze und/oder durch Stimuli von T-lymphozytärem Ursprung, um aktivierte, humane, plasmazytoide, dendritische Zellen zu erhalten;
c) In-Kontakt-Bringen von T-Lymphozyten mit den aktivierten, humanen, plasmazytoiden, dendritischen Zellen aus Schritt: b).

7. Verfahren, um T-Lymphozyten *in vitro* zu aktivieren, gemäß Anspruch 6, **dadurch gekennzeichnet, dass** in Schritt b) die Zellen durch einen Virus und/oder durch IL2 und/oder durch CD40 aktiviert werden.

8. Verfahren, um Verbindungen zu identifizieren, die humane, plasmazytcide, dendritische Zellen aktivieren, **dadurch gekennzeichnet, dass** es die Schritte aufweist:
a) In-Kontakt-Bringen der Verbindung mit den Zellen gemäß einem der Ansprüche 1 bis 2;
b) Detektion der Aktivierung der Zellen.

9. Pharmazeutische Zusammensetzung, **dadurch gekennzeichnet, dass** diese Zellen gemäß einem der Ansprüche 1 bis 2 aufweist.
